# EUROPEAN PATENT SPECIFICATION

(11) **EP 2 203 562 B1**
(45) Date of publication and mention of the grant of the patent: **18.04.2012**
(21) Application number: 08839536.3
(22) Date of filing: 15.10.2008
(51) Int. Cl.: C12P 41/00

(54) **PROCESS FOR THE PREPARATION OF AN ENANTIOMERICALLY AND/OR DIASTEREOMERICALLY ENRICHED ESTER, THIOESTER, ALCOHOL OR THIOL**
VERFAHREN ZUR HERSTELLUNG EINES ENANTIOMERISCH UND/ODER DIASTEREOMERISCH ANGEREICHERTEN ESTERS, THIOESTERS, ALKOHOLS ODER THIOLS
PROCEDE DE PREPARATION D'UN ESTER, THIOESTER, ALCOOL OU THIOL ENANTIOMERIQUEMENT ET/OU DIASTEREOMERIQUEMENT ENRICHI

(30) Priority: 16.10.2007 EP 07020193
(43) Date of publication of application: 07.07.2010
(73) Proprietor: DSM IP Assets B.V., 6411 TE Heerlen (NL)
(72) Inventor: VERZIJL, Gerardus, Karel, Maria, 5855 AR Well (NL); QUAEDFLIEG, Peter, Jan, Leonard, Mario, 6181 KA Elsloo (NL); BROXTERMAN, Quirinus, Bernardus, 6151 JA Munstergeleen (NL)
(74) Representative: Hoogendam, Gerrie Christine
(86) International application number: PCT/EP2008/063915
(87) International publication number: WO 2009/050216

(56) References cited:
- EP-A- 0 415 776
- EDIN ET AL: "Ruthenium- and lipase-catalyzed DYKAT of 1,2-diols: an enantioselective synthesis of syn-1,2-diacetates" TETRAHEDRON: ASYMMETRY, PERGAMON, OXFORD, GB, vol. 17, no. 4, 20 February 2006 (2006-02-20), pages 708-715, XP005401712 ISSN: 0957-4166
- GONZALEZ-SABIN J ET AL: "Chemoenzymatic preparation of optically active beta-amino-cyclohexanols and their application in the enantioselective addition of diethylzinc to benzaldehyde" TETRAHEDRON ASYMMETRY, PERGAMON, OXFORD; GB, vol. 15, no. 8, 19 April 2004 (2004-04-19), pages 1335-1341, XP004501854 ISSN: 0957-4166
- KIRCHNER G ET AL: "RESOLUTION OF RACEMIC MIXTURES VIA LIPASE CATALYSIS IN ORGANIC SOLVENTS" JOURNAL OF THE AMERICAN CHEMICAL SOCIETY, AMERICAN CHEMICAL SOCIETY, WASHINGTON, DC.; US, US, vol. 107, no. 24, 1 January 1985 (1985-01-01), pages 7072-7076, XP002033390 ISSN: 0002-7863

## Description

The invention relates to a process for the preparation of an enantiomerically and/or diastereomerically enriched ester or thioester having at least two adjacent chiral centres. The invention further relates to the use of an ester or thioester or alcohol respectively thiol thus obtained for the preparation of further products.

S. Liu et al. (Angew. Chem. Int. Ed. 2007, 46, 7506) described the synthesis of 1,2-amino alcohols in high enantiomeric excess (e.e). and diastereomeric excess (d.e.) by Ru-catalyzed asymmetric hydrogenation of the racemic ketones but only the cis-diastereomers are accessible using this technology. Further, only tertiary amines are prepared by the described method.

I. Schiffers et al. (J. Org. Chem. 2006, 71, 2320) described the preparation of optically pure trans-2-amino-1-cyclohexanol derivatives based on classical resolution, for instance involving preparation of a diastereoisomeric salt that is precipitated by diastereoisomeric salt precipitation, which has the disadvantage that the theoretically maximum yield is only 50%.

K. Arai et al. (Angew. Chem. Int. Ed. 2007, 46, 955) and F. Carrée et al. (Org. Lett. 2005, 7(6), 1023) describe the synthesis of 1,2-amino alcohols in high e.e. and d.e. by enantioselective ring opening of epoxides by anilines, but this method can only be used for meso-epoxides and only the *trans*-diastereomers are accessible using this method.

It is an objective of the invention to provide an alternative method for preparing an enantiomerically and/or diastereomerically enriched ester or thioester having at least two adjacent chiral centres, in particular a method that overcomes one or more of these drawbacks.

It has now been found possible to prepare an enantiomerically and/or diastereomerically enriched ester or thioester or alcohol respectively thiol having two or more adjacent chiral centres using a Dynamic Kinetic Resolution (DKR), according to the invention. With adjacent is meant that the chiral centres are directly neighbouring carbon atoms, such as the carbon atoms to which hydroxyl groups are attached in 1,2 diols, the carbon atoms to which an amino group respectively a hydroxyl group is attached in 1-amino-2-hydroxy compounds, *etc*..

Accordingly, the invention relates to the preparation of an enantiomerically and/or diastereomerically enriched ester or thioester having at least one pair of two adjacent chiral centres, wherein a mixture of stereoisomers of a secondary alcohol or thiol having a structure comprising a first chiral center forming a secondary alcohol or thiol moiety in the beta position relative to a second chiral center having one hydrogen substituent, is reacted with an acyl donor in the presence of an epimerisation catalyst and a stereoselective acylation catalyst.

The invention also relates to a method for the preparation of an alcohol or thiol from the enantiomerically and/or diastereomerically enriched ester or thioester having at least one pair of two adjacent chiral centres.

An advantage of a process according to the invention is that a product with at least two adjacent chiral centres can be prepared in an enantiomerically and/or diastereomerically enriched form in a one-pot process or a nearly one-pot process, if desired. The invention allows a resolution from a mixture that contains the corresponding stereoisomers in any relative amounts, in a yield that is higher than would be expected on the basis of the relative quantities of the stereoisomers that are initially present.

For the purpose of this invention, with "DKR" is meant a kinetic resolution that is combined with an *in situ* racemisation of the remaining substrate enantiomer. DKR is known for example from Persson et al, J.Am.Chem.Soc. 1999, 121, 1645-1650. The publication describes the conversion of certain racemic secondary alcohols having one chiral center to their enantiomerically enriched esters, but no examples are described for the preparation of enantiomerically and/or diastereomerically enriched chiral esters or chiral secondary alcohols wherein the configuration of at least one pair of two adjacent chiral centres is constituted via DKR.

The inventors found that a stereoselective acylation catalyst can selectively resolve one stereoisomer in high enantiomeric and/or diastereomeric purity from the mixture of stereoisomers. Such a DKR involving two adjacent chiral centres (or a plurality of pairs of adjacent chiral centres) is therefore named "Tandem DKR" (TDKR).

Hereinbelow, the specific alcohol or thiol stereoisomer that is acylated by the acylation catalyst may be referred to as "substrate", unless specified otherwise.

The invention allows a high degree of flexibility with respect of the choice of stereoisomer that is to be enriched.

Further the invention is advantageous in that it allows the preparation of a desired stereoisomer in a high yield.

Advantageously, the present invention allows the in situ formation of the substrate alcohol respectively thiol from the corresponding ketone/thione. This gives the flexibility to employ the ketone/thione or the alcohol respectively thiol or mixtures thereof in a method of the invention.

It is also an advantage of the invention, that the composition of the substrate alcohol respectively thiol with respect to their possible stereoisomers has in principle no effect on the outcome of the TDKR in terms of enantiomeric and/or diastereomeric excess.

The ester respectively thioester or alcohol respectively thiol product obtained in accordance with the invention is enantiomerically enriched and/or diastereomerically enriched. Hereinafter, this will be referred to as "stereo enriched product".

With "enantiomerically enriched" is meant that one of the enantiomers is formed in excess of another enantiomer or even to the exclusion of another enantiomer.

With "diastereomerically enriched" is meant that one of the stereoisomers is formed in excess of another stereoisomer or even to the exclusion of another stereoisomer.

With "diastereomerically enriched and enantiomerically enriched" is meant that one of the stereoisomers in the product is formed in excess of the other stereoisomers or even to the exclusion of one or more of the other stereoisomers.

For the purpose of this invention, with "stereoisomers" are meant compounds which have the same molecular formula and the same sequence of covalently bonded atoms, but different spatial orientations of those atoms.

For the purpose of this invention, with "chiral centre" is meant a grouping of atoms consisting of a central atom and distinguishable covalently bound atoms, such that the interchange of any two of the covalently bound atoms leads to a stereoisomer. Typically, a chiral centre is a carbon atom bearing four different covalently bound atoms.

For the purpose of this invention, with "enantiomers" are meant stereoisomers that are nonsuperimposable complete mirror images of each other.

For the purpose of this invention, with "diastereoisomers" (or "diastereomers") are meant stereoisomers which are not enantiomers.

For the purpose of this invention, with "epimerization" is meant the interconversion of stereoisomers with more than one chiral centre due to configurational lability of one or more chiral centres, which would result in the formation of multiple stereoisomers in ratios that reflect their thermodynamic stabilities.

In accordance with the invention, an epimerisation catalyst is chosen in such a way that it produces - under the conditions applied in the method of the invention - a mixture of stereoisomers that at least contains the particular stereoisomer that is acylated in the method of the invention. For example, if a molecule without a plane of symmetry contains two chiral centres that are subject to epimerisation, four stereoisomers will generally be formed after such an epimerisation process: two diastereomeric pairs of enantiomers. In an exceptional case, however, only one pair of enantiomers may be formed, for instance when the other diastereomeric pair of enantiomers is thermodynamically unlikely to be formed. Suitable epimerisation catalysts will be discussed in further detail below.

In the method of the present invention, the alcohol or thiol moiety of the first chiral center is in the beta-position relative to the second chiral center, i.e. the two chiral carbon atoms are adjacent. The inventors are the first to realize that it is possible to employ a DKR process in which adjacent chiral centres are epimerised under DKR-conditions.

In accordance with the invention, it is possible to epimerise one or more pairs of adjacent chiral centres. The possibility that three consecutive chiral centres are epimerised is also envisaged.

### The substrates

The choice of the substrate secondary alcohol or thiol is determined by the desired product. Also, mixtures of different secondary alcohols or mixtures of different thiols may be used. A secondary alcohol or thiol can, for instance, be represented by the following formula (1), 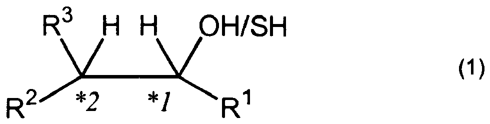 wherein *1 denotes the first chiral centre, wherein *2 denotes the second chiral centre, wherein R¹, R² and R³ are not H.

R¹ and R² may in particular each independently represent an organic moiety, more in particular an optionally substituted linear or branched alkyl group with for instance 1-20 C-atoms, preferably 1-6 C-atoms, an optionally substituted linear or branched alkenyl group with for instance 2-20 C-atoms, preferably 2-6 C-atoms, an optionally substituted linear or branched alkynyl group with for instance 2-20 C-atoms, preferably 2-6 C-atoms, an optionally substituted cycloalkyl group with for instance 3-20 C-atoms, preferably 3-7 C-atoms or an optionally substituted aryl group with for instance 4-20 C-atoms, preferably 5-10 C-atoms. The alkyl, alkenyl, alkynyl, cycloalkyl or aryl group optionally comprises one or more heteroatoms, in particular one or more O, S or N atoms. Alternatively, R¹ and R² may together form an optionally substituted saturated or unsaturated ring structure with for instance 3-20 C-atoms which ring structure may contain one or more heteroatoms, for instance O, S or N.

The alkyl, alkenyl, alkynyl, cycloalkyl or aryl groups of R¹, R², respectively the ring structure may include any substituents that are inert in the reaction system. Suitable substituents are, for example, alkyl groups, aryl groups, alkoxy groups, alkenyl groups, optionally substituted amine groups which are unreactive in the acylation reaction, halogens, nitrile, nitro, acyl, aroyl, carboxyl, carbamoyl or sulphonate groups. The substituents may contain for instance 0-19 C-atoms, particularly 0-10 C-atoms and may comprise one or more heteroatoms, in particular one or more O, S or N atoms.

A particular class of secondary alcohols or secondary thiols is the class where R¹ and R² together form a ring structure, in such a way that the chiral centers *1 and *2 in formula (1) are part of cycloalkyl, cycloalkenyl, heterocycloalkyl, or heterocycloalkenyl moiety.

Preferred examples of secondary alcohols or secondary thiols wherein R¹ and R² together form a ring structure are secondary alcohols or secondary thiols wherein the chiral centers *1 and *2 in formula (1) are part of a cyclopentyl, a cyclohexyl, a cycloheptyl, a pyrolidyl, a piperidinyl or a tetrahydrofuryl moiety.

In an embodiment, substituent R³ in formula (1) may represent an electron withdrawing group. In particular, such a group may be chosen from a primary, secondary or tertiary amine moiety, a secondary alcohol moiety, a secondary thiol moiety, a phosphine moiety or a nitrile moiety, of which a primary amine and an alcohol are preferred. Optionally, such R³ substituent is protected before or during, and deprotected after reacting the secondary alcohol or thiol at the first chiral centre with the acyl donor.

In an embodiment, the invention relates to a process for preparing an enantiomerically and/or diastereomerically enriched ester or thioester having at least one pair of adjacent chiral centres, wherein a mixture of stereoisomers of a secondary alcohol or thiol having a structure comprising a first chiral center forming a secondary alcohol or seconday thiol moiety in the beta position relative to a second chiral center having one hydrogen substituent and not forming a secondary alcohol moiety, is reacted with an acyl donor in the presence of an epimerisation catalyst and a stereoselective acylation catalyst.

M. Edin et al. (Tetrahedron: Asymmetry 2006, 17(4), 708) describe enantioselective transformation of a mixture of four diol isomers to diacetate by lipase-catalyzed regioselective kinetic resolution, in combination with an intramolecular acyl migration and a ruthenium-catalyzed epimerization. Edin et al. teaches that direct enzymatic acylation of the second alcohol moiety takes place to a much lesser extent than enzymatic acylation at the first alcohol moiety. The dynamic kinetic asymmetric transformation process of Edin et al. relies on a faster acyl migration in the syn-diol monoacetate than in the *anti*-diol monoacetate. By contrast in the present invention such a mechanism cannot take place. Surprisingly, enrichment still takes place in the process wherein the second chiral center does not form a secondary alcohol moiety.

In an embodiment, substituent R³ represents an organic moiety, in particular an optionally substituted linear or branched alkyl group with for instance 1-20 C-atoms, preferably 1-6 C-atoms, an optionally substituted linear or branched alkoxy group with for instance 1-20 C-atoms, preferably 1-6 C-atoms, an optionally substituted linear or branched alkenyl group with for instance 2-20 C-atoms, preferably 2-6 C-atoms, an optionally substituted linear or branched alkynyl group with for instance 2-20 C-atoms preferably 2-6 C-atoms or an optionally substituted aryl group. The substituents may contain for instance 4-20 C-atoms, preferably 5-10 C-atoms, and optionally comprise one or more heteroatoms, in particular one or more O, S or N atoms.

The alkyl, alkoxy, alkenyl, and aryl groups of R³ may include any substituents that are inert in the reaction system. Suitable substituents are, for example, alkyl groups, aryl groups, alkoxy groups, alkenyl groups, optionally substituted amine groups which are unreactive in the acylation reaction, halogens, nitrile, nitro, acyl, aroyl, carboxyl, carbamoyl or sulphonate groups. The substituents may contain for instance 0-19 C-atoms, particularly 0-10 C-atoms and may comprise one or more heteroatoms, in particular one or more O, S or N atoms.

In a particular case, the second chiral center of the secondary alcohol or thiol is attached to three carbon atoms. In such case, substituent R³ in formula (1) preferably represents an alkyl or aryl group, more preferably a methyl, ethyl, propyl, butyl, phenyl or benzyl group.

A particular secondary alcohol or thiol which may be used as a substrate in a method of the invention contains two or more pairs of chiral centres each pair comprising a first chiral center forming a secondary alcohol or thiol moiety in the beta position relative to a second chiral center having one hydrogen substituent, i.e. the secondary alcohol or thiol contains at least two pairs of adjacent chiral centres that can be epimerised, for example 1,4-dihydroxy-2-methyl-5-t-butyl-cyclohexane.

### The acyl donors

The substrate alcohol or thiol is reacted with an acyl donor. Preferably, an acyl donor is chosen such that the acyl donor itself does not adversely interfere with the catalytic epimerisation reaction.

Acyl donors that can be used in the process of the present invention are the well known acyl donors as for instance described in Enzyme Catalysis in Organic Synthesis. A comprehensive Handbook, Second, Completely Revised and Enlarged Edition. (Editors: K. Drauz and H. Waldmann), Vol II, 2002, 472, 544, Wiley-VHS, and references cited herein and by U.T. Bornscheuer and R.J. Kazlauskas in the handbook Hydrolases in Organic Synthesis - Regio- and Stereoselective Biotransformations, 1999, Wiley-VCH, chapter 4.2.3. For instance an acyl donor may be selected from the group of carboxylic acid esters, amides and anhydrides, preferably from the group of carboxylic acid esters. Suitable acyl donors in particular include esters of C₁-C₂₀ carboxylic acids and esters of C₁-C₇ alcohols, more in particular esters of C₁-C₂₀ carboxylic acids and C₁-C₇ alcohols. Such acyl donors include acyl donors selected from the group of isopropyl acetate, isopropenyl acetate, isobutyl acetate, vinyl acetate, ethyl acetate, isopropyl laureate, isopropyl butyrate, isopropyl octanoate, isopropyl myristate and isopropenyl laureate.

Preferably, an acyl donor is chosen such that the acyl donor itself has a low volatility under the reaction conditions, that its acyl donor residue has a high volatility and that oxidation of the acyl donor residue is prevented as much as possible under the reaction conditions. Preferred examples of such acyl donors are carboxylic acid esters of an alcohol with 1-4 C-atoms and a carboxylic acid with 4-20 C-atoms. Of these isopropyl butyrate, isopropyl laureate, isopropyl octanoate, isopropyl myristate, isopropenyl acetate and isopropenyl laureate are particularly suitable. Acetone and isopropyl alcohol are preferred examples of acyl donor residues with a high volatility. Preferred acyl donors that produce acetone or isopropyl alcohol are isopropyl laureate, isopropyl octanoate, isopropyl myristate and isopropenyl acetate.

Preferably, the acyl donor residue is removed from the reaction mixture, more preferably it is removed on a continuous basis, for example by preferentially transferring the acyl donor residue to another phase relative to the acyl donor and the other reaction components. This can be achieved by a physical or by a chemical method, or by a combination thereof. Examples of physical methods by which the acyl donor residue can be removed from the phase in which the stereoselective acylation reaction occurs, are selective crystallisation, evaporation, distillation, extraction, complexing to an insoluble complex, absorption and adsorption.

In order to remove the acyl donor residue use can be made of a reduced pressure. The pressure (at a given temperature) is preferably chosen in such a way that the mixture refluxes or is close to refluxing. In addition, the boiling point of a mixture can be lowered by making an azeotropic composition of the mixture. Suitable measures to accomplish this are known in the art. Examples of chemical methods of removal are covalent bonding, chemical derivatisation or enzymatic derivatisation.

### The catalysts - general

In TDKR according to the invention use is made of an epimerisation catalyst and a stereoselective acylation catalyst. These are preferably chosen so that they are mutually compatible, which means that they do not directly or indirectly deactivate each other, at least not to an unacceptable extent. The skilled person can establish by common general knowledge, optionally routine experimentation and using the information disclosed herein what combination of epimerisation catalyst and acylation catalyst is in particular suitable for obtaining a specific stereo-enriched product.

A desired quantity of the acylation catalyst is linked to the quantity of epimerisation catalyst used; the quantity of acylation catalyst is preferably adapted so that the overall reaction continues to proceed efficiently, that is to say, that the acylation reaction proceeds in such a rate relative to the epimerisation reaction, that the stereoisomer acting as the substrate of the acylation catalyst is present in a sufficient amount to allow an acceptable stereoselection by that acylation catalyst. A suitable ratio between epimerisation catalyst and acylation catalyst for a given reaction/catalyst system can routinely be established by experimental means, using common general knowledge and the information disclosed herein. Usually, the amount of acylation catalyst (relative to the epimerisation catalyst) is chosen such that the relative amounts of substrate alcohol or thiol stereoisomers (relative to the total amount of the sterioisomers in the mixture) that are to be acylated is kept sufficiently high in order to let the acylation proceed at a sufficient rate. Further, the amount of acylation catalyst is usually chosen such that the relative amounts of alcohol or thiol stereoisomers that are not involved in the desired acylation reaction is kept sufficiently low such that the enantiomeric excess or diastereiomeric excess of the desired ester or thioester is not adversely affected, or at least not to an unacceptable extent. As a rule of thumb, the higher the enantioselectivity of the acylation catalyst towards the desired ester or thioester, the higher the maximum relative amounts of undesired alcohol or thiol stereoisomers may be, in order to let the reaction proceed well.

### The epimerisation catalysts

The epimerisation catalyst may be in the form of a heterogeneous catalyst or in the form of a homogeneous catalyst.

The epimerisation catalyst may be an enzyme, a metalloenzyme, an organocatalyst or a metal based catalyst.

Preferred epimerisation catalysts include catalysts on the basis of a (transition) metal compound. In particular, the metal may be selected from metals of groups 3, 8, 9, 10, 13 or the lanthanides of the periodic system according to the new IUPAC version as shown in the table printed in the cover of the Handbook of Chemistry and Physics, 82nd edition, CRC press, 2001-2002. Preferred is a metal selected from the group of iron, cobalt, nickel, ruthenium, rhodium, iridium, osmium, palladium, platinum or samarium, including combinations thereof. Of these, a metal selected from the group of ruthenium, iridium, aluminium, samarium and scandium is highly preferred. In a particularly preferred method, the metal is selected from the group of ruthenium, iridium and aluminium. The selection of a preferred metal is dependent on the alcohol or thiol that is used, as will be discussed below.

Suitable transition metal compounds are described for example in Comprehensive Organometallic Chemistry 'The synthesis, Reactions and Structures of Organometallic Compounds' Volumes 1 - 9, Editor: Sir Geoffrey Wilkinson, FRS, deputy editor: F. Gordon A. Stone, FRS, Executive editor Edward W. Abel, preferably volumes 4, 5, 6 and 8 and in Comprehensive Organometallic Chemistry 'A review of the literature 1982 - 1994', Editor-in-chief: Edward W. Abel, Geoffrey Wilkinson, F. Gordon A. Stone, preferably volume 4 (Scandium, Yttrium, Lanthanides and Actinides, and Titanium Group), volume 7 (Iron, Ruthenium, and Osmium), volume 8 (Cobalt, Rhodium, and Iridium), volume 9 (Nickel, Palladium, and Platinum), volume 11 (Main-group Metal Organometallics in Organic Synthesis) and volume 12 (Transition Metal Organometallics in Organic Synthesis).

In particular, the metal-based epimerisation catalyst contains a metal centre, to which may be complexed one or more neutral ligands, for instance one or more compounds to be converted (alcohol, thiol, ketone, thione) or obtained ester or thioester, and to which metal centre is connected an anionic type of ligand. Preferably the catalyst is chosen such that the obtained ester or thioester does not form a complex with the catalyst or at least has a relatively low affinity (low complexation constant) for the metal, compared to the substrate alcohol or thiol. A suitable epimerisation catalyst may in particular be represented by the formula (2)

LₘMⁿₚX_{q}YᵣSₜ. (2)

Each M independently represents a metal in oxidation state n. The integer n is ≥ 1. In particular, M may be selected from the metals identified above.

The integer p represents the number of metal atoms in the catalyst and is ≥ 1. p may have any values of at least 1, for instance p may have a value up to 100. If p > 1 the catalyst is in the form of a cluster. Such clusters may contain many metal atoms, for instance up to 100; in practice often 1-10. Clusters of aluminium alkoxide catalysts are for instance described in (a) "Catalytic applications of aluminum isopropoxide in organic synthesis" by Jerome et al. Chattem Chemicals, Inc., Chattenooga, TN, USA (b) Chemical Industries (Dekker) (2003), 89 (Catalysis of Organic Reactions), 97-114, and references cited therein. The active species of the epimerisation catalyst can be prepared according to methods known in the art for instance as described for MPV catalysts; for instance as described in (a) Yamamoto, H.; Organometallics in Synthesis, A Manual, Second edition (Manfred Schlosser (Editor), 2002, 535-577 John Wiley & Sons Ltd. and references cited herein (b) Eisch, J.J.; Comprehensive Organometallic Chemistry II, a review of the literature 1982-1994 (Wilkinson, G; Stone, G.G.A.; Abel, E.W. ed.), Vol. 1, 1995, 431-502, Pergamon Press, Oxford. If an activation is required, the activation of the epimerisation catalyst may be performed separately or in situ.

L, X, Y and S represent ligands, which ligands can be individual molecules (at least before binding to the metal) or form part of a larger molecule having two or more binding sites for the metal (*i.e*. a polydentate, *e.g*. a bidentate a tridentate, a tetradentate, *etc*.).

Each L independently represents a neutral ligand. L is in particular a ketone or an alcohol and may include one or more compounds to be converted.

The integer m is ≥ 0 and may vary during the process. The integer m may have any value, for instance a value up to 100.

Each X independently represents an anionic ligand. Suitable examples of X are hydride; tetrafluoroborate; halides, in particular Cl⁻ or Br⁻; alkyl groups with *e.g*. 1-12 C-atoms, for example methyl, ethyl, *n*-propyl (ⁿPr) or *i*-butyl (ⁱBu) groups; alkoxy groups with *e.g*. 1-12 C-atoms, for example *n*-pentoxy, *i*-propoxy, *t*-butoxy groups; alkanoate groups with *e.g*. 1-12 C-atoms, for example ethanoate, *n*-butanoate, *n*-pentanoate, *n*-octanoate groups; anions derived from amides, amino acid amides, amino acids, amino amides, amino alcohols or amines; a CN⁻ group; anionic aromatic ligands, in particular cyclopentadienyl (Cp), pentamethyl cyclopentadienyl (Cp*) or indenyl. Preferably the abovementioned alkoxy groups are derived from a secondary alcohol.

The integer q represents the number of ligands X and is ≥ 1, but may have any value, for instance a value up to 100.

Each Y independently represents a so-called spectator ligand, a neutral ligand that has three or more π-electrons available for bonding to the metal, for example an aromatic or an allylic compound, or an olefin with at least two C=C bonds. Examples of aromatic compounds are: benzene, toluene, xylene, cumene, cymene, naphthalene, anisole, chlorobenzene, indene, cyclopentadienyl derivatives, tetraphenyl cyclopentadienone, dihydroindene, tetrahydronaphthalene, gallic acid, benzoic acid and phenylglycine. Examples of olefins are dienes, in particular norbornadiene, 1,5 cyclooctadiene and 1,5-hexadiene.

It is also possible for Y to be covalently bonded to ligand S and/or X.

The integer r represents the number of ligands Y and is ≥ 0, for instance up to 100.

Each S independently represents a neutral ligand. In particular S may be a lone pair donor, that is relatively easy to exchange with other ligands. S may for instance be a phosphine, in particular PPh₃ or PCy₃, a nitrile, a CO or a coordinating solvent molecule, especially tetrahydrofuran (THF), water, acetonitrile, dimethylformamide, dimethyl sulfoxide (DMSO), an alcohol, pyridine, N-methylpyrrolidinone or an amine, in particular a tertiary amine, for example Et₃N. Ligand S may be a single π- or σ- bond donor, such as an olefin, molecular hydrogen or a bridging ligand of type X creating a lone pair bond to the second metal centre in forming the bridge, resulting in a dimeric or polymeric metal compound.

The integer t represents the number of ligands S and is ≥ 0, for instance up to 100.

If necessary, the epimerisation catalyst may be obtained by for example exchanging the neutral ligand S with another ligand S', whereby the metal complex of formula (2) changes into (3)

LₘMⁿₚX_{q}YᵣSₜ₋ᵢS'ᵢ (3)

or by complexing the transition metal compound with a ligand S'. The catalyst on the basis of metal complex (2) and the ligand S' can be added in the form of separate components of which one is the metal complex (2) and the other is the ligand S', or in the form of a complex that already includes S' such as for example formula (3). Examples of S' that lead to suitable epimerisation catalysts are for example a primary or secondary amine, alcohol, diol, amino alcohol, diamine, mono-acylated diamine, O-acylated amino alcohol, mono-tosylated diamine, mono-tosylated amino alcohol, amino acid, amino acid amide, amino-thioether, phosphine, bisphosphine, aminophosphine, preferably an aminoalcohol, monoacylated diamine, monotosylated diamine, amino acid, amino acid amide, amino thioether or an aminophosphine.

For metals of group 8, 9 and 10, a particularly suitable class of ligands is described in EP-A-916637 and in Tetrahedron: Asymmetry 10 (1999) 2045-2061, with the previso that complexation not necessarily takes place with the optically active ligand, but optionally with the racemate corresponding to the optically active ligands described. The ligands are preferably used in quantities from 0.5 to 8 equivalents relative to the metal, in particular from 1 to 3 equivalents. In the case of a bidentate ligand use is preferably made of 0.3 to 8, in particular 0.5 to 3 equivalents.

An example of a particularly suitable class of ligands X and/or S for an epimerisation catalyst based on a metal of group 8, 9 or 10 of the periodic system is the class of amino acid amides of the formula (4). 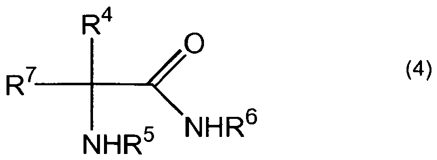 wherein R⁴ and R⁷ each independently represent H or a substituted or unsubstituted alkyl or aryl group with for instance 1-9 C-atoms; R⁵ and R⁶ each independently represent H or a substituted or unsubstituted alkyl or aryl group with for instance 1-9 C-atoms. Two R groups may form a ring, in particular a 5-12 membered ring. For instance, R⁴ and R⁵ may form a ring together, including the N and C atom to which they are bound, R⁵ and R⁶ may form a ring together, including the respective N atoms to which they are bound or R₄ and R₇ may form a ring together.

Examples of a particularly suitable class of ligands for the metals of group 3, 13 and the lanthanides are aryl alcohols, especially bidentate aryl alcohols.

In most cases activation of an epimerisation catalyst, for example catalysts obtained by complexing of the transition metal compound and the ligand, can be effected for example by treating the transition metal compound or the complex of the transition metal compound and the ligand in a separate step with a base, for example KOH, KOtBu, and subsequently isolating it by removing the base and salt which may have formed after adding the base, or by activating the transition metal compound or the complex of the transition metal compound and the ligand in situ, when the acylation/epimerisation takes place, with a mild base, for example a heterogeneous base, in particular KHCO₃ or K₂CO₃, or a homogeneous base, in particular an organic amine, for example triethylamine. It is also possible to activate the transition metal compound with the aid of a reducing agent, for example H₂, formic acid and salts thereof, Zn and NaBH₄.

In particular, if the second chiral center of the secondary alcohol or thiol of formula (1) is attached to three carbon atoms, an epimerisation catalyst is preferred that is based on a metal of group 3, 13, or the group of the lanthanides. In particular preferred is a catalyst based on aluminium, scandium or samarium.

In particular, if R³ in formula (1) represents an amine moiety, a secondary alcohol moiety or a secondary thiol moiety, an epimerisation catalyst is preferred that is based on a metal of group 8, 9 or 10, in particular iridium, ruthenium or rhodium.

In particular, if R³ represents an amine moiety that is protected with an N-protective group, for instance a (substituted) benzyl or a (substituted) benzylidene moiety or if R³ represents an unprotected alcohol moiety, the TDKR can advantageously be carried out with an epimerisation catalyst that is based on a metal of group 8, 9 or 10, in particular iridium, ruthenium or rhodium, preferably iridium.

The relative amount of epimerisation catalyst to be used is not particularly critical and may be selected taking into consideration aspects like its cost, activity, the desired reaction rate, the desired (optical) purity of the product, etc.. The applied relative amount is generally at least 0.001 mol%, in particular at least 0.01 mol%, at least 0.1 mol%, at least 0.5 mol%, or at least 1 mol%, calculated relative to the mixture of stereoisomers of the alcohol or the thiol, at the start of the epimerisation reaction. The applied relative mount is generally less than 100 mol%, in particular less than 50 mol%, less than 20 mol%, less than 10 mol% or less than 5 mol %, based on the mixture of stereoisomers of the alcohol or the thiol.

A catalyst that is based on a metal of group 8, 9 or 10, in particular ruthenium or iridium, preferably is applied in a relative amount of 0.01 to 5 mol%, more in particular of 0.1 to 1 mol% relative to the mixture of stereoisomers of the alcohol or the thiol, at the start of the epimerisation reaction.

A catalyst that is based on a metal of group 3, 13 or a lanthanide, in particular a catalyst that is based on aluminium, scandium or samarium, preferably is applied in a concentration of 0.1 to 50 mol%, in particular of 1 to 20 mol%, more in particular of 5 to 10 mol% relative to the mixture of stereoisomers of the alcohol or the thiol, at the start of the epimerisation reaction.

### The acylation catalysts

The stereoselective conversion of the secondary alcohol or secondary thiol into the corresponding ester or thioester can be carried out with a known stereoselective acylation catalyst, for example as described by Christine E Garrett et al., J.Am.Chem. Soc. 120,(1998) 7479-7483 and references cited therein, and Gregory C. Fu in Chemical innovation/January 2000,3-5.

Preferably the stereoselective conversion of the secondary alcohol or secondary thiol into the corresponding ester or thioester is carried out enzymatically.

Suitable enzymes that can be used in the process according to the invention are for example the known enzymes that have a hydrolytic activity and a high stereoselectivity in the hydrolysis of esters or thioesters to secondary alcohols respectively thiols and that are also active in an organic environment.

A suitable enzyme may in particular be selected from the group of hydrolases (E.C. 3), for example an enzyme with lipase or esterase activity or, when an amide is used as acyl donor, enzymes with amidase activity and esterase or lipase activity may be used.

In a particularly preferred method, the stereoselective acylation catalyst is a hydrolase selected from the group of carboxylic esterases (E.C. 3.1.1), thioester hydrolases (E.C. 3.1.2.) and peptide hydrolases (E.C. 3.4).

The enzyme may in particular originate from *Pseudomonas,* in particular *Pseudomonas fluorescens, Pseudomonas fragi; Burkholderia,* for example *Burkholderia cepacia; Chromobacterium,* in particular *Chromobacterium viscosum; Bacillus,* in particular *Bacillus thermocatenulatus, Bacillus licheniformis; Alcaligenes,* in particular *Alcaligenes faecalis; Aspergillus,* in particular *Aspergillus niger, Candida,* in particular *Candida antarctica, Candida rugosa, Candida lipolytica, Candida cylindracea; Geotrichum,* in particular *Geotrichum candidum; Humicola,* in particular *Humicola lanuginosa; Penicillium,* in particular *Penicillium cyclopium, Penicillium roquefortii, Penicillium camembertii; Rhizomucor,* in particular *Rhizomucorjavanicus, Rhizomucor miehei; Mucor,* in particular *Mucor javanicus; Rhizopus,* in particular *Rhizopus oryzae, Rhizopus arrhizus, Rhizopus delemar, Rhizopus niveus, Rhizopus japonicus, Rhizopus javanicus;* Porcine pancreas lipase, Wheat germ lipase, Bovine pancreas lipase, Pig liver esterase.

Preferably an enzyme originating from *Pseudomonas cepacia, Pseudomonas sp., Burkholderia cepacia, Porcine pancreas, Rhizomucor miehei, Humicola lanuginosa, Candida rugosa* or *Candida antarctica* is used.

Highly preferred is an enzyme selected from the group of *Candida Antarctica* Lipase B (CAL-B), *Burkholderia cepacia* lipase and subtilisin. Of the subtilins, subtilisin Carlsberg is particularly preferred.

When an R-selective enzyme is used, for example from *Candida antarctica,* one of the R-esters is obtained as product. When an S-selective enzyme is used, for example Subtilisin Carlsberg, one of the S-esters is formed. Which of the two possible R- or S-esters is formed depends on the choice of the R- or S-selective enzyme. Such enzymes are commercially available or can be obtained via a generally known technology. In an embodiment, an enzyme that may be used is isolated from a cell from which it originates. In an embodiment, (permeabilised and/or immobilised) cells that have the desired activity, or a homogenate of cells with such an activity may be used. The enzyme can also be used in an immobilised form or in a chemically modified form. Within the framework of the invention it is also possible to use an enzyme originating from a genetically modified microorganism.

### Reaction conditions of the acylations

If desired, the secondary alcohol or thiol that is used as substrate can be formed beforehand from the corresponding ketone or thione in a separate step (that in principle does not need to be stereoselective) with the aid of a reducing ancillary reagent. The reduction preferably is catalysed by the epimerisation catalyst. Preferably a volatile alcohol or a volatile salt of formic acid, preferably its ammonium salt, is used as reducing ancillary reagent, i.e. by non-stereoselective (transfer)hydrogenation. It is also possible to use hydrogen as a reducing reagent.

The mixture of stereoisomers can optionally be formed in situ from the corresponding ketone/thione with the aid of a reducing ancillary reagent. If the alcohol respectively thiol is formed in situ from the ketone/thione, molecular hydrogen or a hydrogen donor is also added as ancillary reagent. As ancillary reagent preferably a secondary alcohol or thiol is added to the reaction mixture that promotes the conversion of the ketone/thione to the alcohol respectively thiol and is not converted by the acylation catalyst. The ancillary reagent is preferably chosen in such a way that (1) it is not also removed from the reaction mixture by the same removal method by which the acyl donor residue is removed, (2) this ancillary reagent is not acylated by the acylation catalyst, and (3) has sufficient reduction potential, relative to the ketone/thione, for the creation of a redox equilibrium. Reducing agents other than alcohols/thiols can of course also be used as ancillary reagents. One skilled in the art can simply determine by experimental means which compounds are suitable for use as ancillary reagents in this reaction system.

As described above, the skilled person can establish by common general knowledge, optionally routine experimentation and using the information disclosed herein what epimerisation-acylation catalyst combination is in particular suitable for his specific system.

In principle, the temperature used for such combination is not critical, as long as the enzyme shows substantial activity. Generally, the temperature may be at least 0 °C, in particular at least 15 °C. A desired maximum temperature depends upon the enzyme. In general such maximum temperature is known in the art, e.g. indicated in a product data sheet in case of a commercially available enzyme, or can be determined routinely based on common general knowledge and the information disclosed herein. Usually, the temperature is 70°C or less, in particular 60°C or less or 50 °C or less. In particular if a thermophilic hydrolytic enzyme is used, the temperature may be chosen relatively high, for instance in the range of 40 to 100 °C, or 40-90°C.

Suitable temperature conditions can be identified for the specific combination of epimerisation catalyst and acylation catalyst by a person skilled in the art through routine experimentation based on common general knowledge and the information disclosed herein. For instance, for subtilisin, in particular subtilisin Carlsberg (e.g. in Alcalase) the temperature may advantageously be in the range of 25-60°C.

The concentration of the alcohol or the thiol is not particularly critical. The reaction can suitably be carried out at relatively high concentrations, for example at a concentration of 0.4 M or more, in particular of 0.8 M or more. Preferably, the concentration of the alcohol or the thiol is 1 M or more, for instance 2 M or more.

In particular, when a relatively polar alcohol or thiol is used in combination with a heterogeneous base to activate an epimerisation catalyst that is based on metals of 8, 9 and 10 of the periodical system, it is favourable that the polarity of the resulting reaction mixture is relatively low. At a low polarity, the occurrence of side-reactions as a result of excessive dissolution of the heterogeneous base is low or may even be absent. The person skilled in the art can determine which conditions meet this requirement by routine experimentation, common general knowledge and the information disclosed herein.

In particular when using an epimerisation catalyst based on a metal from group 3, 13 or the lanthanides, it is desired that the water concentration during the epimerisation is low. Preferably, when using such a catalyst, the epimerisation is carried out in essentially water-free conditions, provided that - if the epimerisation and acylation are carried out in a one-pot process, the acylation catalyst has sufficient activity.

In particular, the water concentration may be less than 4 wt. %, based on the liquid phase, wherein the epimerisation and acylation at least predominantly take place. Advantageously, a method may be carried out in a phase containing less than 2 wt. % water, in particular 1 wt. % or less water, more in particular 0.5 wt. % or less water, 0.2 wt. % or less water, 0.1 wt. % or less water, for instance about 0.05 wt. % or less water or about 0.01 wt. % or less water, whilst still retaining substantial desired acylation catalyst activity and a low, or even undetectable undesired hydrolysis.

For a good acylation catalyst activity, in particular if the acylation catalyst is an enzyme, the presence of a trace of water, e.g. of at least 0.005 wt. % or at least 0.01 wt. %, based on the liquid phase, may be desired, depending on the enzyme. In particular, the water concentration may be at least 0.02 wt. % or at least 0.05 wt. %, based on the liquid phase, for improved acylation activity when using an acylation catalyst for which the presence of (a trace of) water is favourable.

The reaction times are not particularly critical. They generally depend on the relative amounts of the catalyst and reactants, and on the desired conversion.

The product ester obtained may subsequently be isolated from the reaction mixture using a common practice isolation technique, depending on the nature of the ester, for instance extraction, distillation, chromatography or crystallisation. If the product is isolated by crystallisation further stereomeric enrichment may be obtained. If desired, the supernatant (which may contain the alcohol respectively thiol, ester or thioester and/or ketone/thione involved in the reaction) may be recycled to the non stereoselective reduction, for instance (transfer)hydrogenation, or to the conversion of the mixture of the stereoisomers of the alcohol respectively thiol to the enantiomerically and/or diastereomerically enriched ester or thioester. Usually, before recycling solids will be removed from the supernatant and, according to common practice, a purge will be built in in order to prevent built up of impurities. If desired, the ester or thioester in the supernatant can first be hydrolysed to not adversely affect the enantiomeric and diastereomeric excess.

With the process according to the invention an enantiomerically and/or diastereomerically enriched ester or thioester may be obtained with an enantiomeric excess (e.e.) larger than 80%, preferably larger than 90%, more preferably larger than 95%, most preferably larger than 98%, in particular larger than 99%, optionally after (re)crystallization.

With the process according to the invention an enantiomerically and/or diastereomerically enriched ester or thioester can be obtained with an diastereomeric excess (d.e.) larger than 80%, preferably larger than 90%, more preferably larger than 95%, most preferably larger than 98%, in particular larger than 99%, optionally after (re)crystallization.

The enantiomerically and/or diastereomerically enriched ester or thioester obtained can subsequently be used as such, or be converted, e.g., in a corresponding alcohol or thiol.

### Applications of the esters/thioesters

The ester or thioester may be used in the preparation of for example a liquid crystal, an agrochemical, a food or feed additive, fragrance material, cosmetical ingredient or a pharmaceutical ingredient, optionally after converting the ester or thioester into the corresponding secondary alcohol or secondary thiol as will be discussed below in further detail. Suitable method to convert an ester or thioester into an alcohol respectively thiol are generally known in the art and include hydrolysis, transesterification, amidation and alcoholysis.

### Reaction condition conditions for the preparation of the alcohol or thiol

If the alcohol respectively thiol is the desired product, the enantiomerically and/or diastereomerically enriched ester or thioester may subsequently converted by a known procedure into the corresponding enantiomerically and/or diastereomerically enriched alcohol respectively thiol. This can for example be effected by means of a conversion catalysed by an acid, base or enzyme. When a stereoselective enzyme is used, the enantiomeric or diastereomeric excess of the product alcohol respectively thiol can be increased. When the stereoselective acylation according to the invention has been carried out with the aid of an enzyme, the same enzyme can very suitably be used for the conversion of the enantiomerically and/or diastereomerically enriched ester or thioester into the enantiomerically and/or diastereomerically enriched alcohol respectively thiol. When the ultimate goal is the preparation of the alcohol respectively thiol, the acyl donor can be freely chosen in such a way that the physical or chemical properties of the acyl donor and the acyl donor residue are suitable for the removal of the acyl donor residue and the treatment of the reaction mixture.

In a process according to the invention enantiomerically and/or diastereomerically enriched alcohols/thiols with an enantiomeric excess larger than 95%, preferably larger than 98%, more preferably larger than 99% can be obtained, optionally after recrystallization and/or hydrolysis with the aid of a stereoselective enzyme.

In a process according to the invention enantiomerically and/or diastereomerically enriched alcohols/thiols with an diastereomeric excess larger than 95%, preferably larger than 98%, more preferably larger than 99% can be obtained, optionally after recrystallization and/or conversion of the ester or thioester into the alcohol respectively thiols with the aid of a stereoselective enzyme, in particular a stereoselective hydrolase.

### Applications of the alcohols/thiols

The invention also relates to the preparation of an enantiomerically and/or diastereomerically enriched alcohol respectively thiol from the enantiomerically and/or diastereomerically enriched ester or thioester obtained. The alcohol respectively thiol thus obtained may be used in the preparation of for example a liquid crystal, an agrochemical, a food or feed additive, fragrance material, cosmetical or a pharmaceutical ingredient.

The invention will be elucidated on the basis of the examples.

### EXAMPLES

### General

Unless stated otherwise, chemicals were obtained from commercial sources and used without further purification. All materials directly used in the Tandem DKR reactions were dried under vacuum in a Schlenk tube.

In case reaction mixtures were inertisized this entailed the application of vacuum at gentle reflux at room temperature followed by purging with nitrogen (5 times).

### Example I - Tandem DKR of a mixture of racemic trans and cis-2-methylcyclohexanol

### Preparation of the epimerisation catalyst 1

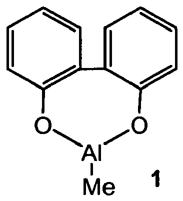

The used catalyst 1 (from the Meerwein-Pondorff-Verley series) was prepared from AlMe₃ and 2,2'-biphenol. In a 50 mL Schlenk tube equipped with a magnetic stirring bar the internal standard hexamethylbenzene (56.2 mg, 0.346 mmol) was dissolved in toluene (20 mL) and the solution was inertisized. Subsequently, a 2 M solution of AlMe₃ in toluene (0.5 mL, 1 mmol) and 2,2'-biphenol (186.4 mg, 1 mmol) were added resulting in the liberation of methane gas. The reaction mixture was heated at 70°C for 15 min and cooled to ambient temperature.

### Tandem DKR

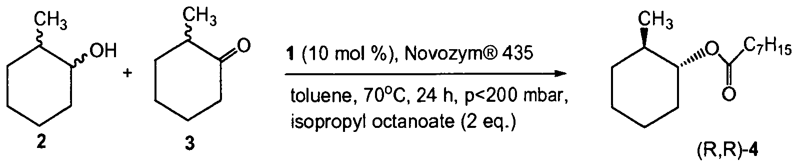

To remove traces of water, Novozym^{®} 435 (5 g) was stirred for 1 h at 70°C in a solution of isopropenyl acetate (5 g, 0.05 mol) in toluene (100 mL). Subsequently, the solid enzyme was isolated by filtration and dried under an atmosphere of N₂.

In the catalyst solution (prepared as described above) was dissolved a mixture of trans and cis (trans/cis = 4.0) 2-methylcyclohexanol 2 (0.57 g, 5 mmol) resulting in methane evolution. After the evolution of gas had ceased, 2-methylcyclohexanone 3 (5 mmol) and isopropyl octanoate (20 mmol, 2 equiv.) were added. Subsequently, dried Novozym^{®} 435 (100 mg) was added and the temperature was slowly increased to 70°C. At a constant temperature of 70°C, the pressure was slowly reduced over a period of 6 h to approximately 180 mbar (in order to remove isopropanol and acetone). The reaction was conducted for another 18 h and monitored by chiral GC using a CP-Chirasil-Dex CB column (length 25 m) at 250 bar with a flow of 3.8 mL/min using an FID detector. The following temperature gradient was used: 100°C for 13 min, 100→150°C by 20°C/min, 150°C for 8.5 minutes. Hexamethylbenzene was used as the internal standard. Retention times: (S)-3 (3.29 min), (R)-3 (3.37 min), trans-2 (5.23 min), cis-2 (5.98 min), (S,S)-4 (22.28 min), (R,R)-4 (22.44 min), (R,S)-4 (22.15 min), (S,R)-4 (22.02 min), hexamethylbenzene (17.46 min).

The amounts of 2 and 3 were calculated by comparing the peak areas (relative to the internal standard) of the 4 stereoisomers of 2 and the 2 stereoisomers of 3 with the values at the start of the Tandem DKR. The amount of 4 was calculated from the conversion of 2 and 3. The results are given in the table below.

| t (h) | e.e (%) 3 | e.e.(%) 4 | conversion 2+3 | amount (R,R)-4 | trans/cis 2 | trans/cis 4 |
|---|---|---|---|---|---|---|
| 0 | 1 | | 0 | 0.0 | 4.0 | |
| 2 | 9 | > 99 | 11 | 11 | 3.8 | |
| 4 | 13 | 98 | 19 | 19 | 3.9 | 118 |
| 6 | 16 | 98 | 27 | 26 | 3.9 | 102 |
| 8 | 18 | 98 | 33 | 33 | 3.9 | 102 |
| 24 | 78 | 97 | 75 | 73 | 4.8 | 55 |

As can be concluded from these results the mixture of 4 diastereomers of 2 and racemic 3 has been transformed to essentially one stereomer of 4 in high conversion (73%).

### Example II - Tandem DKR of trans-N-benzyl-2-amino-cyclohexanol (5)

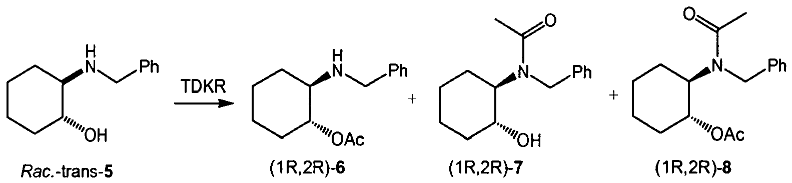

In a 100 mL three-necked round bottom flask equipped with thermometer, distillation unit and magnetic stirring bar were dissolved [RuCl₂cymene]₂ (30.6 mg, 0.05 mmol) and (R,S)-2-phenyl-2-aminopropionamide (32.8 mg, 0.2 mmol) in toluene (20 mL). The mixture was heated at 70°C for 15 min under an atmosphere of N₂ resulting in a partially dissolved yellow complex. Subsequently, racemic trans-*N*-benzyl-2-aminocyclohexanol (5) (prepared according to Synthetic Comm., 31 (21), 2001, 3295-3302; 2.05 g, 10 mmol) was added resulting in complete dissolution of the complex.

Then, isopropenyl acetate (2.0 g, 20 mmol), Novozym^{®} 435 (150 mg) and dry K₂CO₃ (1 g) were added. For GC analysis, hexamethylbenzene (80 mg, 0.494 mmol) was added as the internal standard. The reaction mixture was inertisized and heated at 70°C for 2 h at atmospheric pressure. Under these conditions the acetone formed from the acetylation with isopropenyl acetate accumulated in the reaction mixture. Subsequently, the pressure was slowly reduced to approximately 260 mbar (to remove the acetone). The reaction was continued for 26 h at 260 mbar and 70°C and sampled.

The conversion was determined by GC using a WCOT Fused Silica column (length 25 m, internal diameter 0.32 mm) with a CP-Sil5-CB coating (DF 1.2 µm) using an FID detector. The following temperature gradient was used: 50°C for 3 min, 50→250°C by 15°C/min, 250°C for 15 min. Hexamethylbenzene was used as the internal standard. The retention times were: hexamethyl benzene (14.26 min), 5 (16.95 min), cis-6 (17.68 min), trans-6 (17.85 min), 7 (20.74 min), 8 (22.18 min).

The e.e. of trans-6 was determined by chiral GC. To this end an aliquot of approximately 20 µL of the reaction mixture was diluted in CH₂Cl₂ (1 mL) and injected on coating CP-Chirasil-Dex CB column (length 25 m, DF = 0.25). The following temperature gradient was used: 120°C for 30 min, 120→200°C by 20°C/min, 200°C for 6 min. Retention times were: (S,S)-6 31.6 min, (R,R)-6 31.96 min.

It was observed that 91 % of the starting material had mainly been converted to the corresponding product (1 R,2R)-6 (e.e. > 99%). Also some 7 and 8 had been formed.

The product was filtered over SiO₂, the solids washed with ethyl acetate (25 mL) and the combined filtrates concentrated *in vacuo* giving crude product (2.34 g).

### Example III - Tandem DKR of trans-N-benzyl-2-amino-cyclohexanol (5)

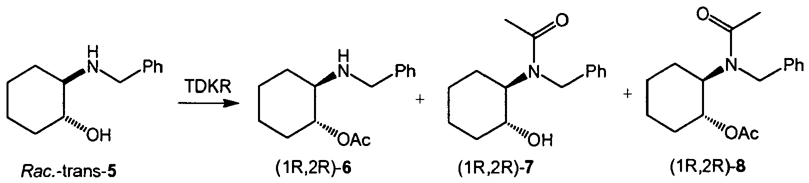

In a 50 mL three-necked round bottom flask equipped with thermometer, distillation unit and magnetic stirring bar were dissolved [RuCl₂cymene]₂ (15.3 mg, 0.025 mmol) and (R,S)-2-phenyl-2-aminopropionamide (16.4 mg, 0.1 mmol) in toluene (20 mL). The mixture was heated at 70°C for 15 min resulting in a partially dissolved yellow precipitated complex. Addition of isopropanol (3 mL) completely dissolved the complex and the resulting homogeneous yellow solution was stirred for 30 min at 70°C. Subsequently, hexamethylbenzene (80 mg; internal standard for GC) and racemic trans-*N*-benzyl-2-aminocyclohexanol (5) (prepared according to Synthetic comm., 31 (21), 2001, 3295-3302; 1.03 g, 5 mmol) were dissolved in the reaction mixture and toluene/isopropanol were completely distilled at 70°C under reduced pressure. The resulting residue was redissolved in toluene (10 mL) and a sample analysed by GC.

Subsequently, isopropenyl acetate (10 mmol), Novozym^{®} 435 (40 mg) and K₂CO₃ (0.5 g, 3.6 mmol) were added. The reaction mixture was inertisized and the pressure slowly decreased to 265 mbar. The reaction mixture was stirred for 18 h at 70°C and 265 mbar. Subsequently, fresh Novozym^{®} 435 (20 mg) was added and the reaction mixture stirred for another 48 h at 70°C and 265 mbar. The resulting final mixture was analysed by GC. See the table below.

| t (h) | amount (%) | | | | |
|---|---|---|---|---|---|
| | 5 | trans-6 | cis-6 | 7 | 8 |
| 66 | 16 | 72 | 5.9 | 7.6 | 2 |

The reaction mixture was filtered over SiO₂ and the solids washed with EtOAc (25 mL). The combined filtrate was concentrated in vacuo and the residue subjected to hydrolysis in 6 N aqeous HCl (50 mL) at 90°C for 24 h. This furnished a mixture of trans- and cis-5 in 84% and 62% e.e. respectively.

The e.e. of 5 and 7 was determined by chiral HPLC on a Chiralpak AD column (250 x 4.6 mm) using *n*-heptane/methanol/ethanol/diethylamine (90/3/2/0.05 v/v/v/v) as the eluent with a column temperature of 50°C and a flow of 0.8 mL/min. Detection was performed with UV (210 nm). The injection volume was 5 µL (sample solution in eluent). Retention times: (1R,2S)-5 and (1S, 2R)-5 (both cis-5 enantiomers) 7.27 and 8.04 min; (1S,2S)-5 and (1R,2R)-5 (both trans-5 enantiomers) 9.09 and 9.80 min; (1S,2S)-7 and (1R,2R)-7 (both trans-7 enantiomers) 17.84 and 22.97 min.

### Example IV - Tandem DKR of trans-N-benzyl-2-amino-cyclohexanol (5)

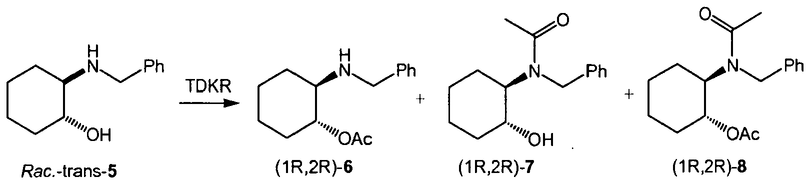

In a 50 mL three-necked round bottom flask equipped with thermometer, distillation unit and magnetic stirring bar were dissolved [RuCl₂cymene]₂ (15.3 mg, 0.025 mmol), (R,S)-2-phenyl-2-aminopropionamide (16.4 mg, 0.1 mmol) and hexamethylbenzene (80 mg; internal standard) in toluene (17 mL) and isopropanol (3 mL). The mixture was heated at 70°C for 15 min under an atmosphere of nitrogen giving a clear yellow solution. Subsequently, racemic trans-*N*-benzyl-2-aminocyclohexanol (5) (prepared according to Synthetic comm., 31 (21), 2001, 3295-3302; 1.03 g, 5 mmol) was dissolved in the reaction mixture and toluene/isopropanol were completely distilled at 70°C under reduced pressure. The resulting residue was redissolved in toluene (10 mL) and a sample analysed by GC.

Subsequently, isopropenyl acetate (10 mmol), Novozym^{®} 435 (50 mg) and K₂CO₃ (0.5 g, 3.6 mmol) were added. The reaction mixture was inertisized and stirred for 3 h at 70°C and atmospheric pressure. In this period of time, the colour changed from yellow to orange. The accumulated acetone was removed after slow reduction of the pressure (to approximately 300 mbar) causing the colour to change to deep red. The reaction mixture was stirred for 5 h at 70°C and 300 mbar and an additional amount of isopropenyl acetate (10 mmol) was added. The reaction was stirred for another 18 h at 70°C and 300 mbar and analyzed by GC (as described in example II). The results are given in the table below. The e.e. of 5 and 7 was determined by chiral HPLC (as described in example III).

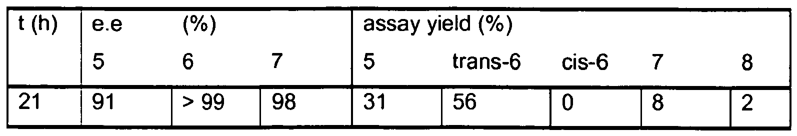

### Example V - Tandem DKR of trans-N-benzylidene-2-amino-cyclohexanol (9)

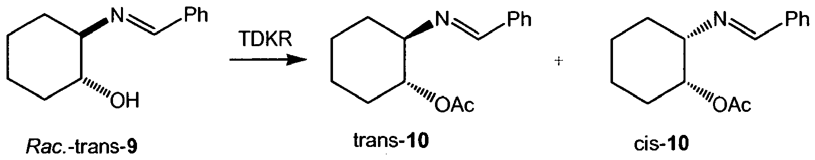

### Tandem DKR

In a 250 mL three-necked round bottom flask equipped with thermometer, distillation unit and magnetic stirring bar were dissolved [IrCp*Cl₂]₂ (dichloro(pentamethylcyclopentadienyl) iridium (III) dimer; 200 mg, 0.25 mmol) and 2-methyl-2-aminopropionamide (62 mg, 0.61 mmol) in acetonitrile (50 mL). After heating the mixture at 70°C for 15 min, K₂CO₃ (5.4 g, 0.039 mol) was added and the mixture was heated for another 15 min at 70°C during which time the colour changed from yellow to red. The acetonitrile was completely distilled off at reduced pressure and toluene (60 mL) was added to the residue giving a heterogeneous mixture. Subsequently, hexamethylbenzene (0.4 g) was added as the internal standard. Toluene (10 mL) was partly distilled off at 70°C under reduced pressure to remove traces of acetonitrile. Racemic trans-9 (0.1 mol) was dissolved in the resulting solution and a sample of the mixture was analyzed by GC.

The reaction mixture was heated for 2 h at 70°C (epimerisation) and then isopropenyl acetate (20 g, 0.2 mol) and Novozym^{®} 435 (250 mg) were added. In order to remove the formed acetone the pressure was slowly reduced to 165 mbar with the temperature being kept at 70°C. After 5, 23 and 26 h 250 mg, 100 mg and 100 mg portions, respectively, of fresh Novozym^{®} 435 were added. After 29 h, an additional amount of isopropenyl acetate (10 g, 0.1 mol) was added. The course of the reaction was monitored by GC and HPLC (see below). The results are given in the table below.

| t | e.e. (%) | | Assay yield (%) | | | | Remarks |
|---|---|---|---|---|---|---|---|
| (h) | Trans-9 | Cis-9 | Trans-9 | Cis-9 | Trans-10 | Cis-10 | |
| 0 | | | 99 | 1 | | | |
| 2 | | | 78 | 19 | | | Epimerisation |
| 0 | | | 78 | 19 | | | TDKR |
| 5 | 48 | 29 | 32 | 15 | 49 | 1 | |
| 23 | 1 | 35 | 12 | 5 | 76 | 2 | |
| 30 | 35 | 46 | | | | | |
| 46 | | | 5 | 1 | 88 | 2 | |

### GC and HPLC analysis

The course of the reaction was monitored by GC using hexamethylbenzene as the internal standard using the GC conditions as in example II. Retention times: hexamethyl benzene (14.22 min), cis-9 (16.69 min), trans-9 (16.79 min), cis-10 (17.44 min), trans-10 (17.59 min).

The e.e. of cis- and trans-9 was determined by chiral HPLC using 3 columns in series: two 50 x 4.6 mm I.D. Chiralcel OD columns and one 250 x 4.6 mm I.D. Lichrosphere Diol column. The eluent was *n*-heptane/2-propanol 90/10 (v/v), the column temperatures 40°C and the flow 1.0 mL/min. UV (210 nm) was used for detection. Samples were prepared by dilution of 20 µl of the reaction mixture in eluent (1 mL). Injection volume was 5 µL. Retention times were: (1 R,2S)-9 and (1 S,2R)-9 (both cis-9 enantiomers) 12.2 and 15.1 min; (1R,2R)-9 and (1S,2S)-9 (both trans-9 enantiomers) 13.3 and 15.3 min.

E.e. determination of the isolated HCl salt of (R,R)-2-aminocyclohexanol was performed according to the same chiral HPLC method but using the following sample preparation: the solid product (76 mg) was heated in a mixture of acetonitrile (2 mL), K₂CO₃ (276 mg) and benzaldehyde (42 mg) for 15 min at 70°C. Of the resulting solution 20 µL was dissolved in eluent (1 mL).

### Product isolation and deprotection

The K₂CO₃ and the enzyme were removed from the reaction mixture by filtration and to the filtrate was added water (20 mL) and concentrated aq. HCl (37 wt%) (10 mL) and the mixture was heated at 70°C during several hours. The hydrolysis reaction was monitored by GC and conducted until all intermediate O-acetyl-2-amino cyclohexanol was transformed to the end product 2-aminocyclohexanol. The aqueous layer was washed with toluene (3 x 50 mL) and concentrated in vacuo to give a brown viscous oil residue (15.5 g). Traces of water were removed by azeotropic distillation using isopropanol (50 mL) giving a brown solid (13.5 g) which was stirred in acetonitrile (100 mL) giving a white to grey solid residue. The solid was isolated by filtration, washed with acetonitrile (3 x 25 mL) and dried under N₂. This gave the desired HCl salt of (R,R)-2-aminocyclohexanol as an off-white solid (10.42 g, 0.069 mol) corresponding to 69% yield based on racemic trans-9. The e.e. was > 99%.

### Example VI - Tandem DKR of trans-N-benzylidene-2-amino-cyclohexanol (9)

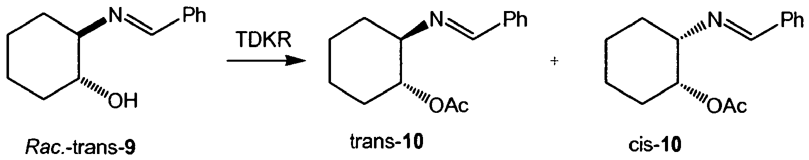

### Tandem DKR

In a 100 mL round bottom flask of equipped with thermometer, distillation unit and magnetic stirring bar were dissolved [IrCp*Cl₂]₂ (19.9 mg, 0.25 mmol), 2-methyl-2-aminopropionamide (7.1 mg, 0.07 mmol) in isopropanol (5 mL) and toluene (10 mL) and the solution was heated at 70°C for 15 min. The volatiles were removed under reduced pressure giving a yellow solid to which, at ambient temperature, were added hexamethyl benzene (internal standard; 80 mg), toluene (10 mL), isopropenyl acetate (20 mmol), racemic trans-9 (5 mmol), K₂CO₃ (0.5 g, 0.0036 mol) and lipase AK (200 mg). The reaction mixture was inertisized and heated at 70°C for 1 h at atmospheric pressure followed by continuously reducing the pressure to 270 mbar. After 22 h, another portion of lipase AK (100 mg) was added to the reaction mixture and the reaction continued for 8 h.

The reaction mixture was cooled to ambient temperature and the solids removed by filtration over SiO₂ and washed with toluene (3 x 10 mL). The combined yellow filtrates were concentrated in vacuo to furnish a residue (1.15 g) which was redissolved in methanol (10 mL) and 1 N aq. HCl (10 mL) and refluxed for 17 h. After concentration in vacuo the residue was redissolved in water (10 mL) and washed with toluene (2 x 10 mL). The aqueous layer was concentrated in vacuo and traces of water were removed by azeotropic distillation with isopropanol (2 x 10 mL). The resulting residue was stirred in acetonitrile (10 mL) resulting in crystallization. The solids were isolated by filtration and dried with nitrogen giving slightly yellow crystalline (R,R)-2-amino-cyclohexanol product (0.51 g) corresponding to 67% yield based on racemic trans-9. The e.e. was > 99 % and the trans/cis ratio was 17.

The reaction was monitored by chiral HPLC and GC according to example V. Sample preparation and subsequent e.e. determination of the isolated product were also performed as in example V. The results are given in the table below.

| t | e.e. (%) | | assay yield (%) | | | | Remarks |
|---|---|---|---|---|---|---|---|
| (h) | Trans-9 | Cis-9 | Trans-9 | Cis-9 | Trans-10 | Cis-10 | |
| | | | | 2 | 18 | | Atmospheric |
| 1 | 20 | 56 | 79 | | | | pressure |
| | | | | 8 | 71 | 4 | Reaction at |
| 22 | 1 | 87 | 15 | | | | 270 mbar |
| 30 | 2 | 72 | 7 | 5 | 83 | 5 | |

## Claims

1. Process for preparing an enantiomerically and/or diastereomerically enriched ester or thioester having at least one pair of two adjacent chiral centres, wherein a mixture of stereoisomers of a secondary alcohol or thiol having a structure comprising a first chiral center forming a secondary alcohol or secondary thiol moiety in the beta position relative to a second chiral center having one hydrogen substituent and not forming a secondary alcohol moiety, is reacted with an acyl donor in the presence of an epimerisation catalyst and a stereoselective acylation catalyst.

2. Process according to claim 1, wherein the secondary alcohol or thiol contains two or more pairs of adjacent chiral centres, each pair comprising a first chiral center forming a secondary alcohol or thiol moiety in the beta position relative to a second chiral center having one hydrogen substituent as defined in claim 1.

3. Process according to claim 1 or 2, wherein the second chiral center forms an amine moiety, an alkyl moiety or a secondary thiol moiety.

4. Process according to claim 3, wherein the amine moiety or secondary thiol moiety of the second chiral center is protected before or during, and deprotected after reacting the secondary alcohol or thiol at the first chiral centre with the acyl donor.

5. Process according to claim 1 or 2, wherein the second chiral center is attached to three carbon atoms.

6. Process according to any one of claims 1-5, wherein the first chiral centre and the second chiral center are part of a cycloalkyl, cycloalkenyl, heterocycloalkyl, or heterocycloalkenyl moiety.

7. Process according to claim 6, wherein the first and second chiral centers are part of a cyclopentyl, cyclohexyl, cycloheptyl, pyrolidyl, piperidinyl or tetrahydrofuryl moiety.

8. Process according to any one of claims 1-7, wherein the epimerisation catalyst is based on a metal of group 3, 8, 9, 10, 13 or the group of lanthanides of the periodic table, the metal being in an oxidation state equal to or higher than 1.

9. Process according to claim 8, wherein the epimerisation catalyst is based on a metal selected from the group of ruthenium, iridium, aluminium, samarium and scandium.

10. Process according to any one of claims 1-9, wherein the stereoselective acylation catalyst is a hydrolase, preferably a hydrolase selected from the group of carboxylic esterases, thioester hydrolases and peptide hydrolases, more preferably selected from *Candida Antarctica* Lipase B (CAL-B), *Burkholderia cepacia* lipase and subtilisin.

11. A process according to any one of claims 1-10, wherein the mixture of stereoisomers is prepared in situ by reducing a ketone or thione having a chiral center with one hydrogen substituent, which chiral centre is in the alpha position relative to the carbonyl or the thiocarbonyl moiety.

12. Process for preparing an enantiomerically and/or diastereomerically enriched secondary alcohol or thiol having at least adjacent two chiral centres, wherein the enantiomerically and/or diastereomerically enriched ester or thioester obtained in a process according to any one of claims 1-11 is converted into the secondary alcohol or thiol, preferably by hydrolysis, transesterification or amidation, more preferably by hydrolysis.

13. Process according to claim 12, wherein the enantiomerically and/or diastereomerically enriched ester or thioester is stereoselectively converted.

## Patentansprüche

1. Verfahren zur Herstellung eines enantiomeren-und/oder diastereomerenangereicherten Esters oder Thioesters mit wenigstens einem Paar aus zwei benachbarten chiralen Zentren, wobei man ein Stereoisomerengemisch eines sekundären Alkohols oder Thiols mit einer Struktur, die ein erstes chirales Zentrum, das eine Sekundärer-Alkohol-oder Sekundäres-Thiolgruppierung bildet, in der Beta-Stellung relativ zu einem zweiten chiralen Zentrum, das einen Wasserstoffsubstituenten aufweist und keine Sekundärer-Alkoholgruppierung bildet, umfasst, mit einem Acyldonor in Gegenwart eines Epimerisierungskatalysators und eines Katalysators für die stereoselektive Acylierung umsetzt.

2. Verfahren nach Anspruch 1, wobei der sekundäre Alkohol oder das sekundäre Thiol zwei oder mehr Paare benachbarter chiraler Zentren enthält, wobei jedes Paar ein erstes chirales Zentrum, das eine Sekundärer-Alkohol- oder Sekundäres-Thiolgruppierung bildet, in der Beta-Stellung relativ zu einem zweiten chiralen Zentrum, das einen Wasserstoffsubstituenten aufweist, wie in Anspruch 1 definiert, umfasst.

3. Verfahren nach Anspruch 1 oder 2, wobei das zweite chirale Zentrum eine Amingruppierung, eine Alkylgruppierung oder eine Sekundäres-Thiolgruppierung bildet.

4. Verfahren nach Anspruch 3, wobei die Amingruppierung oder Sekundäres-Thiolgruppierung des zweiten chiralen Zentrums vor oder während der Umsetzung des sekundären Alkohols oder Thiols am ersten chiralen Zentrum mit dem Acyldonor geschützt und nach der Umsetzung entschützt wird.

5. Verfahren nach Anspruch 1 oder 2, wobei das zweite chirale Zentrum an drei Kohlenstoffatome gebunden ist.

6. Verfahren nach einem der Ansprüche 1-5, wobei das erste chirale Zentrum und das zweite chirale Zentrum Teil einer Cycloalkyl-, Cycloalkenyl-, Heterocycloalkyl- oder Heterocycloalkenylgruppierung sind.

7. Verfahren nach Anspruch 6, wobei das erste und das zweite chirale Zentrum Teil einer Cyclopentyl-, Cyclohexyl-, Cycloheptyl-, Pyrolidyl-, Piperidinyl- oder Tetrahydrofurylgruppierung sind.

8. Verfahren nach einem der Ansprüche 1-7, wobei der Epimerisierungskatalysator auf einem Metall der Gruppe 3, 8, 9, 10, 13 oder der Lanthanidengruppe des Periodensystems basiert, wobei sich das Metall in einem Oxidationszustand gleich oder höher als 1 befindet.

9. Verfahren nach Anspruch 8, wobei der Epimerisierungskatalysator auf einem aus der Gruppe Ruthenium, Iridium, Aluminium, Samarium und Scandium ausgewählten Metall basiert.

10. Verfahren nach einem der Ansprüche 1-9, wobei es sich bei dem Katalysator für die stereoselektive Acylierung um eine Hydrolase, vorzugsweise eine aus der Gruppe Carbonsäure-Esterasen, Thioester-Hydrolasen und Peptid-Hydrolasen, besonders bevorzugt aus *Candida antarctica*-Lipase B (CAL-B), *Burkholderia cepacia*-Lipase und Subtilisin ausgewählte Hydrolase handelt.

11. Verfahren nach einem der Ansprüche 1-10, wobei das Stereoisomerengemisch in situ hergestellt wird, indem man ein Keton oder Thion, das ein chirales Zentrum mit einem Wasserstoffsubstituenten aufweist, reduziert, wobei sich das chirale Zentrum in der Alpha-Stellung relativ zu der Carbonyl- oder der Thiocarbonylgruppierung befindet.

12. Verfahren zur Herstellung eines enantiomeren-und/oder diastereomerenangereicherten sekundären Alkohols oder Thiols mit wenigstens zwei benachbarten chiralen Zentren, wobei der in einem Verfahren nach einem der Ansprüche 1-11 erhaltene enantiomeren- und/oder diastereomerenangereicherte Ester oder Thioester vorzugsweise durch Hydrolyse, Umesterung oder Amidierung, besonders bevorzugt durch Hydrolyse in den sekundären Alkohol bzw. das sekundäre Thiol umgewandelt wird.

13. Verfahren nach Anspruch 12, wobei der enantiomeren- und/oder diastereomerenangereicherte Ester oder Thioester stereoselektiv umgewandelt wird.

## Revendications

1. Procédé de préparation d'un ester ou thioester énantiomériquement et/ou diastéréomériquement enrichi ayant au moins une paire de deux centres chiraux adjacents, où un mélange de stéréoisomères d'un alcool ou thiol secondaire ayant une structure comprenant un premier centre chiral formant un motif d'alcool secondaire ou de thiol secondaire en position bêta par rapport à un deuxième centre chiral ayant un substituant hydrogène et ne formant pas de motif d'alcool secondaire, est réagi avec un donneur d'acyle en présence d'un catalyseur d'épimérisation et d'un catalyseur d'acylation stéréosélective.

2. Procédé selon la revendication 1, dans lequel l'alcool ou thiol secondaire contient deux, ou plus, paires de centres chiraux adjacents, chaque paire comprenant un premier centre chiral formant un motif d'alcool ou de thiol secondaire en position bêta par rapport à un deuxième centre chiral ayant un substituant hydrogène tel que défini selon la revendication 1.

3. Procédé selon la revendication 1 ou 2, dans lequel le deuxième centre chiral forme un motif amine, un motif alkyle ou un motif thiol secondaire.

4. Procédé selon la revendication 3, dans lequel le motif amine ou le motif thiol secondaire du deuxième centre chiral est protégé avant ou pendant, et déprotégé après, la réaction de l'alcool ou thiol secondaire au niveau du premier centre chiral avec le donneur d'acyle.

5. Procédé selon la revendication 1 ou 2, dans lequel le deuxième centre chiral est fixé à trois atomes de carbone.

6. Procédé selon l'une quelconque des revendications 1-5, dans lequel le premier centre chiral et le deuxième centre chiral font partie d'un motif cycloalkyle, cycloalcényle, hétérocycloalkyle ou hétérocycloalcényle.

7. Procédé selon la revendication 6, dans lequel le premier et le deuxième centre chiral font partie d'un motif cyclopentyle, cyclohexyle, cycloheptyle, pyrrolidyle, pipéridinyle ou tétrahydrofuryle.

8. Procédé selon l'une quelconque des revendications 1-7, dans lequel le catalyseur d'épimérisation est à base d'un métal du groupe 3, 8, 9, 10, 13 ou du groupe des lanthanides du Tableau Périodique, le métal étant dans un état d'oxydation égal ou supérieur à 1.

9. Procédé selon la revendication 8, dans lequel le catalyseur d'épimérisation est à base d'un métal choisi dans le groupe constitué par le ruthénium, l'iridium, l'aluminium, le samarium et le scandium.

10. Procédé selon l'une quelconque des revendications 1-9, dans lequel le catalyseur d'acylation stéréosélective est une hydrolase, préférablement une hydrolase choisie dans le groupe constitué par les estérases carboxyliques, les hydrolases spécifiques des thioesters, et les hydrolases spécifiques des peptides, plus préférablement choisie parmi la lipase B de *Candida antarctica* (CAL-B), la lipase de *Burkholderia cepacia* et la subtilisine.

11. Procédé selon l'une quelconque des revendications 1-10, dans lequel le mélange de stéréoisomères est préparé *in situ* par la réduction d'une cétone ou d'une thione ayant un centre chiral avec un substituant hydrogène, lequel centre chiral est en position alpha par rapport au motif carbonyle ou thiocarbonyle.

12. Procédé de préparation d'un alcool ou thiol secondaire énantiomériquement et/ou diastéréomériquement enrichi ayant au moins deux centres chiraux adjacents, dans lequel l'ester ou thioester énantiomériquement et/ou diastéréomériquement enrichi obtenu dans un procédé selon l'une quelconque des revendications 1-11 est converti en alcool ou thiol secondaire, préférablement par hydrolyse, transestérification ou amidation, plus préférablement par hydrolyse.

13. Procédé selon la revendication 12, dans lequel l'ester ou thioester énantiomériquement et/ou diastéréomériquement enrichi est converti de manière stéréosélective.
